# EUROPEAN PATENT APPLICATION

(11) **EP 1 733 744 A1**
(43) Date of publication of application: **20.12.2006**
(21) Application number: 05013151.5
(22) Date of filing: 17.06.2005
(51) Int. Cl.: A61K 49/00

(54) **Method, dye and medicament for staining the internal limiting membrane and/or the capsule of an eye**

(71) Applicant: Ludwig-Maximilians-Universität München, 80333 München (DE)
(72) Inventor: Haritoglou, Christos, PD Dr. med., 80469 München (DE)
(74) Representative: Pfenning, Meinig & Partner GbR

(57) **Abstract**

The present invention concerns a method of staining the internal limiting membrane and/or the lens capsule of the eye as well as dyes and medicaments suitable for this method. Such dyes, medicaments and methods are needed in ophthalmic surgery, in particular in macular and/or cataract surgery.

According to the inventive method at least one of a fluorescent dye, Lightgreen SF, Copper-phtalocyaninetetrasulfonic acid tetrasodium salt, E68, Bromophenolblue and Chicagoblue is administered to the eye.

## Description

The present invention concerns a method of staining the internal limiting membrane and/or the lens capsule of the eye as well as dyes and medicaments suitable for this method. Such dyes, medicaments and methods are needed in ophthalmic surgery, in particular in macular and/or cataract surgery.

At present, vital dyes are used to assist ophthalmic surgery both in the anterior as well as in the posterior segment. Especially in macular surgery, dye-assisted vitrectomy allowed a better intraoperative visualization of the vitreoretinal interface. Two dyes are commonly used in ophthalmic surgery:
Trypan blue was first suggested to stain the lens capsule to assist capsulorrhexis and for the evaluation of the corneal endothelium of donor tissue before performing penetrating keratoplasty. It is now also available for staining of epiretinal tissue and the internal limiting membrane (ILM) in macular pucker surgery. While toxic effects were described in an animal model, no dye related complications have been described in humans. Nevertheless, carcinogenic and teratogenic properties of trypan blue have been described in animal models.

Indocyanine green ICG has a long history as a diagnostic tool for the imaging of choroidal perfusion during angiography. Intraocularly, it was used to stain the lens capsule in mature cataract. Recently, ICG was the first dye introduced for ILM staining during macular surgery. However, the known light absorbing and photooxidative properties of ICG made this dye also applicable for the destruction of tumor cells in vivo and in vitro and for photodynamic therapy at the level of the choriocapillaris. While the intravenous application of ICG is still considered very safe, concerns of dye related toxicicty when applied intraocularly emerged after several reports on adverse effects observed experimentally and clinically.

It is thus the aim of the present invention to provide dyes which differentially stain the internal limiting membrane and/or the lens capsule of the eye and have only a small or no toxic effect at all on the retinal pigment epithelium (RPE). Further, a medicament containing these dyes, their use and a method of staining the internal limiting membrane and/or the lens capsule should be provided.

This problem is solved by the membrane according to claim 1, the medicament according to claim 7, the dyes according to claim 9 and their use according to claim 11. Further improvements of said method, dyes, medicaments and use are provided in the respective dependent claims.

The inventive dyes all stain lens capsules as well as epiretinal membranes in a differential manner thus allowing to differentiate the lens capsule and/or the epiretinal membrane from surrounding tissue, e.g. during macular pucker surgery. All these dyes show a long wavelength absorption maximum within the range of 586 to 634 nm lying in the visible region. Most important and different to the dyes used in the prior art and others not claimed, these dyes showed no relevant toxicity or no toxicity at all, making them suitable for staining tissue in the eye.

The invention thus identifies dyes with satisfying staining characteristics and revealing no relevant or no detectable toxicity.

The inventive dyes light green SF, E68, Bromophenol blue and Chicago blue are defined as follows.

### Light green SF:

C₃₇H₃₄N₂Na₂O₉S₃

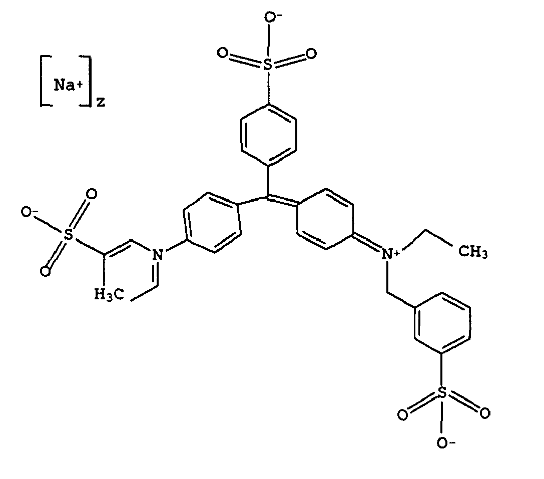

### E68:

Copperphtalocyanine-3,4',4'',4'''-tetrasulphonic acid tetrasodium salt

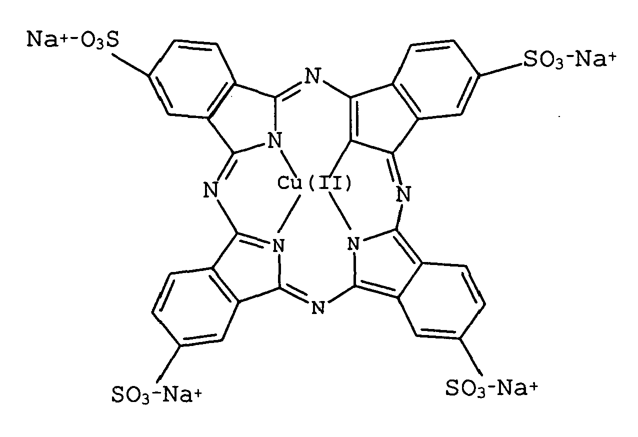

### Bromophenole blue:

C₁₉H₁₀Br₄O₅S

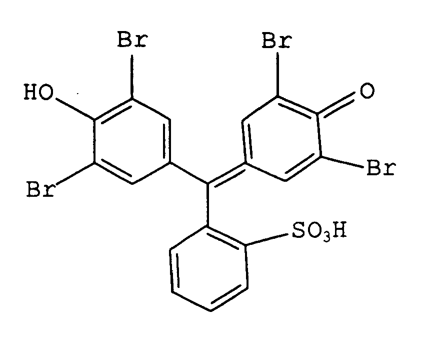

### Chicago blue:

C₃₄H₂₄N₆Na₄O₁₆S₄

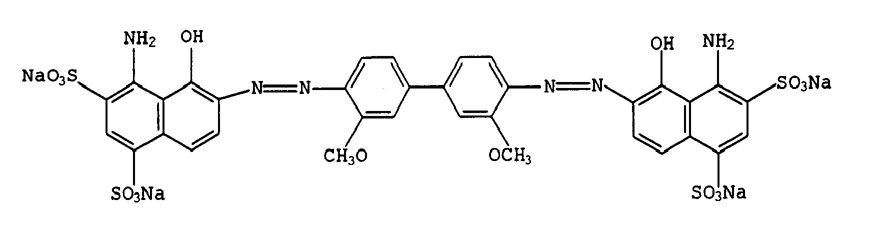

In the following results are shown on the use of the dyes light green SF (LGSF), E68, Bromophenole blue (BPB) and Chicago blue (CB).

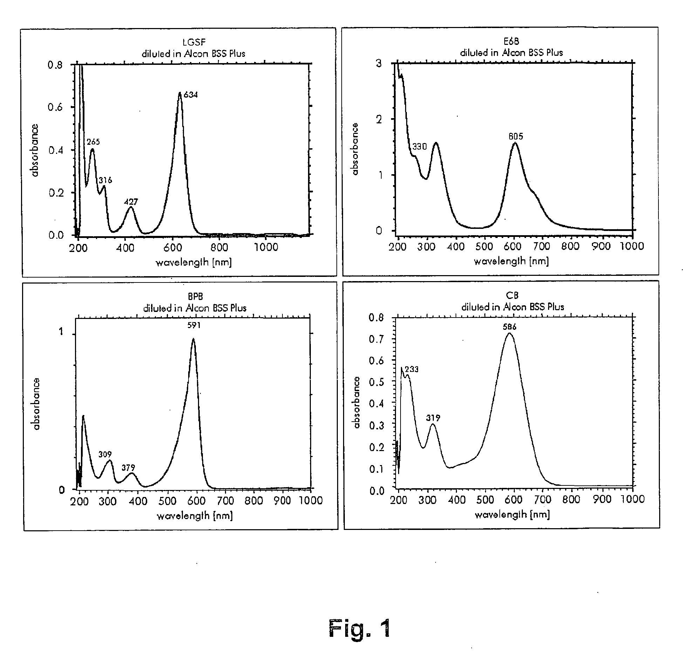

- Fig. 1: shows the light absorbing properties of Light green yellowish SF (LG SF), E68, Bromophenol blue (BPB) and Chicago blue (CB). The maximum peak of absorption varied between 586 and 634nm. Most dyes showed no relevant light absorption between 400 and 500 nm and beyond 700 nm,

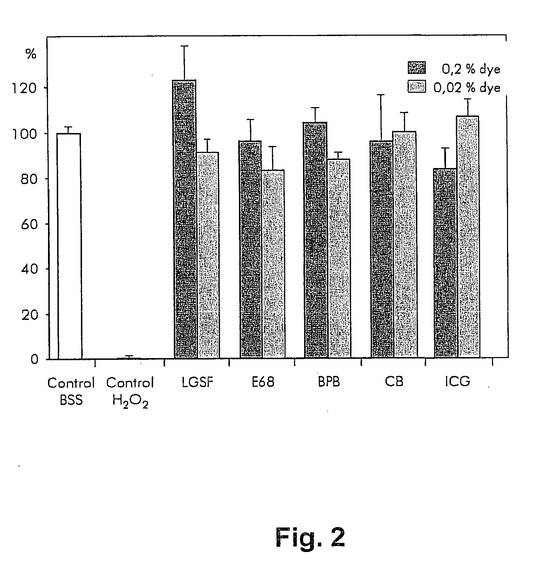

- Fig. 2: shows the viability of ARPE-19 cells measured after treatment with the investigated dyes measured by a colorimetric test (MTT). Tests were performed in triplicate and repeated three times. ARPE-19 cells of the same passage incubated with equal volumes of BSS without addition of dyes served as the control. Results were expressed as the mean percentage of control survival. Data are the mean of results in three experiments, each performed in triplicate. Error bars, SEM. (Light green yellowish SF (LG SF), E68, Bromophenol blue (BPB), Chicago blue (CB)). Indocyanine green (ICG) as a commercially available dye was also tested. No statistically relevant differences between both concentrations (0.2% and 0.02%) were observed. BSS plus alone and hydrogen-peroxide (H₂O₂,/200µl/ml) served as controls.

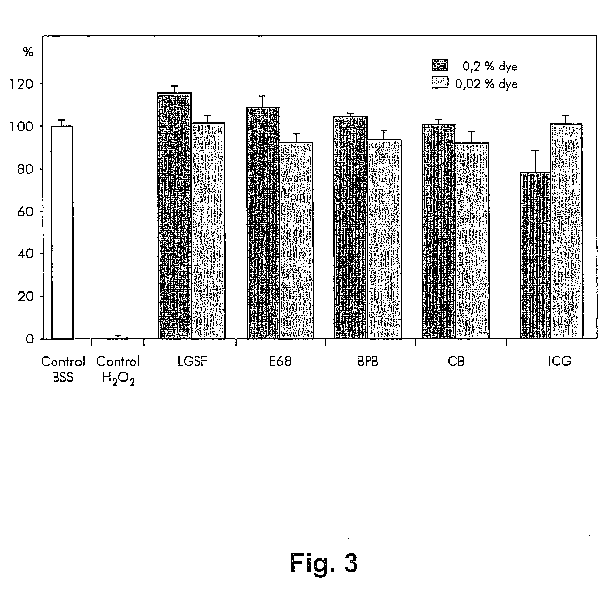

- Fig. 3: shows the viability of retinal pigment epithelium (RPE) cells measured after treatment of cells with the investigated dyes measured by a colorimetric test (MTT). RPE cells of the same passage incubated with equal volumes of BSS without addition of dyes served as the control. Results were expressed as the mean percentage of control survival. Data are the mean of results in three experiments, each performed in triplicate. Error bars, SEM. (Light green yellowish SF (LG SF), E68, Bromophenol blue (BPB), Chicago blue (CB)). Indocyanine green (ICG) as a commercially available dye was also tested. The differences between both concentrations were statistically significant only for Light green yellowish SF (LG SF, p ≤ 0.05) and Indocyanine green (ICG, p ≤ 0.05). BSS plus alone and hydrogenperoxide (H₂O₂,/200µl/ml) served as controls.

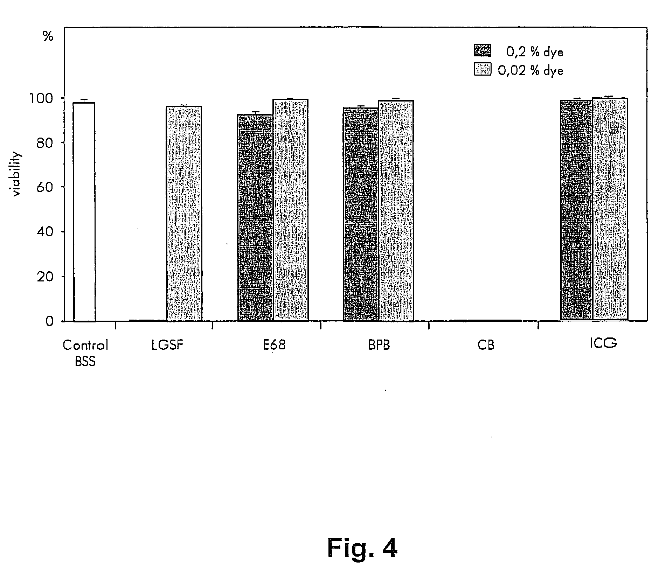

- Fig. 4: shows the quantification of the effect of the tested dyes (0.2% and 0.02%) on the number of nonviable cells in cultures of ARPE 19 cells. The percentage of dead cells was scored by counting at least 1400 cells in fluorescence photomicrographs of representative fields. Data (mean ± SEM) are based on the sampling of 6 to 10 photomicrographs per condition in three independent experiments performed in duplicate. (Light green yellowish SF (LG SF), E68, Bromophenol blue (BPB), Chicago blue (CB)). Indocyanine green (ICG, 0.5% and 0.05%) as a commercially available dye was also tested.

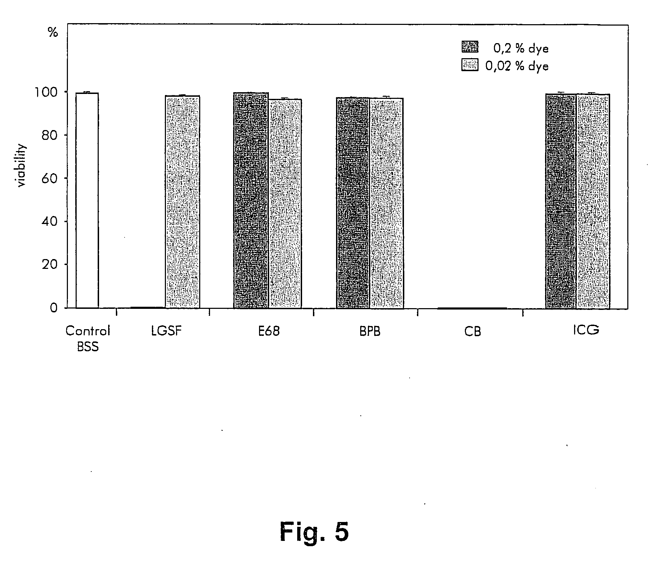

- Fig. 5: shows the quantification of the effect of the tested dyes (0.2% and 0.02%) on the number of nonviable cells in cultures of primary RPE cells. The percentage of dead cells was scored by counting at least 1400 cells in fluorescence photomicrographs of representative fields. Data (mean ± SEM) are based on the sampling of 6 to 10 photomicrographs per condition in three independent experiments performed in duplicate. (Light green yellowish SF (LG SF), E68, Bromophenol blue (BPB), Chicago blue (CB)). Indocyanine green (ICG, 0.5% and 0.05%) as a commercially available dye was also tested.

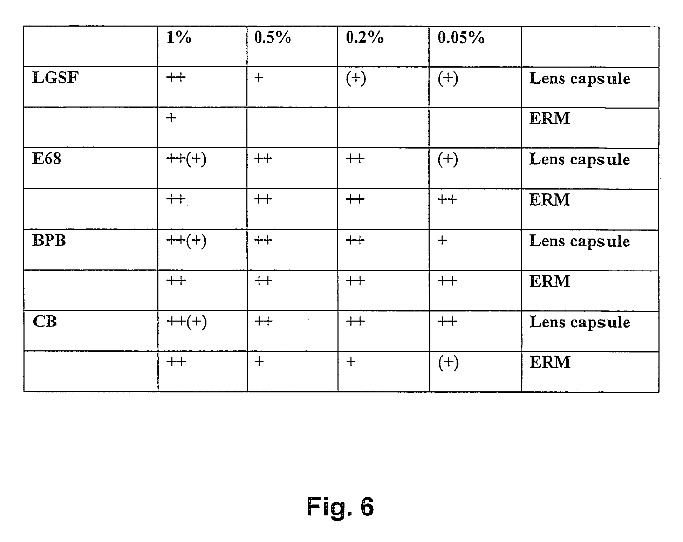

- Fig. 6: shows the results of the grading of the staining effects in lens capsule and epiretinal membrane (ERM) using different dyes and dye concentrations. The staining effects was graded as excellent (+++), good (++), fair (+), absent (-). Light green yellowish SF (LG SF), E68, Bromophenol blue (BPB) and Chicago blue (CB). The first row refers to the results of staining of lens capsule, the second of epiretinal tissue. The staining effect was evaluated against a white background.

For the following example, methods according to the following section were used.

### Dye selection and evaluation of staining characteristics

The following four dyes were examined as examples for the inventive dyes in the present study: Light green yellowish SF (LG SF), E68, Bromophenol blue (BPB) and Chicago blue (CB). ICG (Pulsion, Munich, Germany) was used as a reference. All dyes were dissolved and diluted with balanced salt solution (BSS plus; Alcon Laboratories Inc., Fort Worth, TX) and concentrations of 1.0, 0.5, 0.2 and 0.05% were obtained. Dry ICG powder was first dissolved with sterile water provided by the manufacturer resulting in a 0.5% solution and then further diluted using BSS plus to a concentration of 0.05%. The dyes were then used to stain lens capsule and epiretinal membranes removed during intraocular surgery. Immediately after removal, the material was placed on a glass slide and covered with a few drops of the dye'. After one minute, the dye was carefully removed by irrigation using BSS plus. Lens capsule and epiretinal tissue was then evaluated macroscopically and using light microscopy. The staining effect was subjectively graded as "excellent, good, fair or absent" by one unmasked person (S.P) and photographs were taken.

Additionally, the staining characteristics of the lens capsule in enucleated porcine eyes with a post-mortem time of nine hours were evaluated. The dyes were injected into the air-filled anterior chamber and removed by irrigation after one minute. Then, the cornea was removed and a capsulorrhexis was performed with a bent needle. All procedures were taped on video.

### Light absorbing properties

Light absorption was measured in 0.05 % solutions, with BSS plus as a solvent medium. Light absorption was measured instantly after preparation of a stock solution of the dye using a UV/VIS/NIR Spectrometer (Lambda 900, Perkin Elmer) between 200 and 1000 nm. In contrast to ICG the solubility of the other dyes in BSS plus solution is much higher (> 0.05 % dye).

### Evaluation of dye toxicity

### Human RPE Cell Culture

RPE cells from five human donors were obtained from the Eye Bank of the Ludwig-Maximilians-University (Munich, Germany) and were prepared as described in Alge CS, Priglinger SG, Neubauer AS, Kampik A, Zillig M, Bloemendal H, Welge-Lussen U., Retinal pigment epithelium is protected against apoptosis by alphaB-crystallin. Invest Ophthalmol Vis Sci 2002; 43: 3575-82. In brief, whole eyes were thoroughly cleansed in 0.9 % NaCl solution, immersed in 5% polyvinyl pyrrolidone iodine, and rinsed again in the sodium-chloride solution. The anterior segment from each donor eye was removed and the posterior poles were examined with the aid of a binocular stereomicroscope to confirm the absence of gross retinal disease. Next, the neural retinas were carefully peeled away from the RPE-choroid-sclera using fine forceps. The eye cup was rinsed with Ca²⁺ and Mg²⁺ -free Hank's balanced salt solution, and filled with 0.25% trypsin (GIBCO, Karlsruhe, Germany) for 30 min at 37°C. The trypsin was carefully aspirated and replaced with Dulbecco's modified eagles medium (DMEM, Biochrom, Berlin, Germany) supplemented with 20 % fetal calf serum (FCS, Biochrom). Using a pipette, the media was gently agitated, releasing the RPE into the media by avoiding damage to Bruch's membrane. The RPE cell solution was transferred to a 50 ml flask (Falcon, Wiesbaden, Germany) containing 20 ml of DMEM (Biochrom) supplemented with 20 % FCS (Biochrom) and maintained at 37° C and 5% carbon dioxide. Epithelial origin was confirmed by immunohistochemical staining for cytokeratin using a pan-cytokeratin antibody (Sigma). The cells were tested and found free of contaminating macrophages (anti-CD11; Sigma) and endothelial cells (anti-von Willbrand factor, Sigma) (data not shown). After having grown to confluency (100%), primary RPE cells were subcultured and maintained in DMEM (Biochrom) supplemented with 10 % FCS (Biochrom) at 37°C and 5 % carbon dioxide. Primary RPE of passage 3-6 and ARPE-19 cells were used for experiments.

ARPE-19 cells, a human retinal pigment epithelial cell line (42), were purchased from ATCC (Manassas, VA, USA) and grown in a 1:1 mixture of Dulbecco's modified Eagles medium and Ham's F12 medium (DMEM/Ham's F12, Biochrom), supplemented with 10 % FCS (Biochrom).

### MTT Assay

For exposure of dyes RPE and ARPE-19 cells were kept for 24 hours under serum free conditions. After washing cells three times with PBS, cells were incubated for 10 minutes with 300 µl of BSS plus containing 0.2 % or 0.02 % of dye, respectively. This rather long exposure time is reasonable as it increases the chance of detecting toxicity, but nevertheless does not mimic the current clinical use of ICG or trypan-blue or the likely use of any new dye. The dye was then removed by carefully rinsing cells with BSS plus three times. After 24 hours incubation with serum containing media the cell proliferation assay was performed. RPE cells (p3-6) were seeded in 24-well plates and exposed to two concentrations (0.2 and 0.02%) of dyes. BSS plus alone and H₂O₂, (200µL/mL) served as controls.

The tetrazolium dye-reduction assay (MTT; 3-[4,5-dimethylthiazol-2-yl]-2,5-diphenyl tetrazolium bromide;) was used to determine cell survival rate. The MTT test was performed as described in the literature by Mosmann T., Rapid colorimetric assay for cellular growth and survival: application to proliferation and cytotoxicity assays. J Immunol Methods 1983; 65: 55-63. The medium was removed, cells were washed with PBS, and 1000 µL/well MTT solution (1.5 mL MTT stock, 2 mg/mL in PBS, plus 28.5 mL DMEM) was added. RPE cells were incubated at 37°C for 1 hour. The formazan crystals that formed were dissolved by the addition of dimethyl sulfoxide (DMSO; 1000 µL/well). Absorption was measured by a scanning multiwell spectrophotometer at 550 nm (Molecular Probes, Garching, Germany). Results from the wells were expressed as the mean percentage of control proliferation. Experiments were performed in triplicate and repeated three times. RPE and ARPE-19 cells cells of the same passage incubated with BSS without addition of dyes served as control. Statistical comparison between dye concentrations was performed with SPSS (Mann-Whitney-U-Test).

The MTT test as performed in this study is a well established test for the assessment of cell viability, but relies on colorimetric measurement of a blue (550 nm) formazan reaction product. This color overlaps with the absorption spectra of some of the dyes tested. Therefore, control experiments were performed in order to check for potential interferences of residual dye with the assay. Cell monolayers were treated with dyes as per the experiments described above but absorbance readings were performed without prior application of MTT. No differences after dye treatment compared to BSS controls were found. Experiments were performed in triplicate and repeated three times.

### Life-Dead Assay

Confluent RPE and ARPE-19 cells were prepared and treated as described above. Cell viability was quantified based on a two-color fluorescence assay in which the nuclei of nonviable cells appear red because of staining by the membrane-impermeable dye propidium iodide (Sigma), whereas the nuclei of all cells were stained with the membrane-permeable dye Hoechst 33342 (Intergen, Purchase, NY). Confluent cultures of RPE cells growing on coverslips in 24-well tissue culture plates were exposed to 0.2% and 0.02% Light green yellowish SF (LG SF), E68, Bromophenol blue (BPB), Chicago blue (CB) as described for MTT assays. For evaluation of cell viability, cells were washed in PBS and incubated with 2.0 µg/mL propidium iodide and 1.0 µg/mL Hoechst 33342 for 20 minutes at 37°C. Subsequently, cells were analyzed with an epifluorescence microscope (Axioskop; Zeiss, Göttingen, Germany). The labeled nuclei were then counted in fluorescence photomicrographs, and dead cells were expressed as a percentage of total nuclei in the field. The data are based on counts from three experiments performed in duplicate wells, with three to five documented representative fields per well. RPE and ARPE-19 cells of the same passage incubated with BSS without addition of dyes and H₂O₂ (200µL/mL) served as control.

### Evaluation of staining characteristics

The staining effect in removed lens capsule material and ERM varied between the different dyes and dye concentrations applied. Light green yellowish (LGSF) did not stain lens capsule sufficiently using concentrations of 0.5 % or less and only a concentration of 1 % provided a weak staining of ERM. Lower concentrations were therefore not tested using LGSF. The other dyes revealed excellent to good staining effects both in lens capsule and ERM even at a lower concentration of 0.2 %. The grading of the staining effect of each dye and dye concentration is shown in fig. 6. In porcine eyes, the lens capsule could be stained well with Bromphenol blue (BPB), Chicago blue (CB) and E68, while LGSF provided weak staining effects. In general, the contrast seen in porcine eyes was less pronounced, as the staining effect was evaluated against the background of the clear porcine lens. Intraoperatively, dyes are used to assist capsulorhexis predominantly in mature cataracts, where one should expect a much better contrast.

### Light absorbing properties

The light absorbing properties and peaks of maximum absorption of dye concentrations of 0.05 % were variable. The long wavelength maximum peak of absorption was in the range 527 - 655 nm (see fig. 1). Except Light green SF yellowish (LGSF) no dye showed relevant light absorption between 400 and 500 nm. Absorption maxima beyond 700 nm of any of the investigated dyes were not found.

### Evaluation of dye toxicity

### MTT Assay

Compared to BSS plus without addition of any dye serving as a control, the four novel inventive dyes (Light green SF (LG SF); E68; Bromphenol blue (BPB); Chicago blue (CB)) showed no significant impact on cell survival of ARPE-19 cells neither in a concentration of 0.2 nor of 0.02 % (see fig. 2). Additionally, no influence on cell survival of primary RPE cells was observed after exposition to LGSF, E68, BPB and CB at concentrations of 0.2 and 0.02 % (fig. 3). The differences between both concentrations were statistically significant only for Light green SF (LG SF, p ≤ 0.05) and Indocyanine green (ICG, p ≤ 0.05) in primary RPE cells.

### Life-Dead Assay

When the viability of RPE cells was tested by labeling of the nuclei of nonviable cells with propidium iodide 24 hours after treatment of cells, two dyes (Light green yellowish (LGSF) and Chicago blue (CB)) were identified to significantly affect cell viability compared to controls treated with BSS plus alone. After treatment with CB, this effect was seen both in cultures of ARPE-19 and primary RPE cells in concentrations of 0.2% and 0.02%. However, in comparison to the 0.2% dye solution, 0.02% LG SF appeared to be far less toxic. E68, Bromophenol blue (BPB) and BSS plus (control) did not affect cell survival.

### Summary

The use of vital dyes to intraoperatively stain ocular tissue such as the lens capsule or epiretinal membranes or the ILM potentially facilitate ophthalmic surgery. The introduction especially of ICG to assist macular surgery was initially met with great enthusiasm in the ophthalmic community as it appeared to make intraocular surgery safer and more controllable by visualizing and facilitating the removal of the ILM during surgery for tractive maculopathies such as macular pucker or macular holes. Additionally, staining of the vitreoretinal interface could open the door also for the less experienced surgeon to follow the principle of ILM removal in macular surgery. However, as there are observations indicating potential dye related toxicity under yet not completely understood circumstances, the use of ICG has become a controversial subject among surgeons. The question whether ICG should be considered a "toxic adjunct" is currently under investigation. No in-vivo or in-vitro safety studies concerning the intraocular use of ICG preceded the clinical, intraocular application of ICG.

Given the known chemical properties of ICG and the current debate on potential toxic effects of ICG after intraocular application, the following considerations might be of interest when choosing and evaluating novel dyes:
a) The dye should exhibit a large absorption coefficient. Large extinction coefficients allow for the injection a significantly lower amount of the dye. As a consequence the formation of aggregates is suppressed at low dye concentrations.
b) The dye should show a high solubility in water and BSS (which is used as an irrigation solution during surgery), but should not show a concentration dependence. In most cases this effect is accompanied by a dramatic shift of the absorption maximum and / or with the appearance of new absorption bands.
c) The material should have a high photochemical stability. That means irreversible photoreaction in the first excited singlet state. S1 or triplet state T1, as described for the photosensitizer ICG, should be avoided.
d) The triplet quantum yield should be as low as possible avoiding singlet oxygen formation that can lead to an irreversible oxidation of the ILM / tissue or to photodegradation of the dye itself. On the other hand the lifetime of singlet oxygen both in water and in the solid state is very short.
e) The staining material should have a minimal dark toxicity.
f) The dye should exhibit very good adsorption properties towards the target tissue.

It is evident from the above brief description that all requirements can hardly be met and compromises should be found.

With the present example different dyes of both cationic and anionic character from different dye classes have been shown to be highly suitable for ophthalmic surgery according to the above criteria. These dyes have large absorption coefficients (5 x 10⁴ -20 x 10⁵ L mol⁻¹ cm⁻¹). All compounds show a high solubility in water and BSS solution. Their photostability and dark stability in water or BSS solution are in some cases much better than the one of ICG (data not shown). The position of the long wavelength maximum of Light green SF yellowish, E68, Bromophenol blue, Chicago blue is not influenced by the dye concentration (measured up to a content of 0.05% dye).

## Claims

1. Method of staining the internal limiting membrane (membrana limitans interna, ILM) and/or the lense capsule of the eye,
**characterized in that**
at least one of a fluorescent dye, Lightgreen SF, Copper-phtalocyanine-tetrasulfonic acid tetrasodium salt, E68, Bromophenolblue and Chicagoblue is administered to the eye.

2. Method according to the preceding claim, **characterized in that** at least one of a fluorescent dye, Lightgreen SF, Copper phtalocyanine-tetrasulfonic acid tetrasodium salt, E68, Bromophenolblue and Chicagoblue is administered by intraocular injection.

3. Method according to one of the preceding claims,
**characterized in that** at least one of a fluorescent dye, Lightgreen SF, Copper phtalocyanine-tetrasulfonic acid tetrasodium salt, E68, Bromophenolblue and Chicagoblue is administered to the internal limiting membrane and/or the lense capsule.

4. Method according to one of the preceding claims,
**characterized in that** at least one of a fluorescent dye, Lightgreen SF, Copper phtalocyanine-tetrasulfonic acid tetrasodium salt, E68, Bromophenolblue and Chicagoblue is administered to a mammal.

5. Method or use according to the preceding claim,
**characterized in that** the mammal is a human.

6. Method according to one of the preceding claims,
**characterized in that** the fluorescent dye is Rhodamine.

7. Medicament comprising at least one of a fluorescent dye, Lightgreen SF, Copper phtalocyanine-tetrasulfonic acid tetrasodium salt, E68, Bromophenolblue and Chicagoblue.

8. Medicament according to the preceding claim,
**characterized in that** the fluorescent dye is Rhodamine.

9. A fluorescent dye, Lightgreen SF, Copper phtalocyanine-tetrasulfonic acid tetrasodium salt, E68, Bromophenolblue and/or Chicagoblue for use in medicine.

10. A fluorescent dye according to the preceding claim, **characterized in that** the fluorescent dye is Rhodamine.

11. Use of at least one of a fluorescent dye, Lightgreen SF, E68, Bromophenolblue and Chicagoblue for the manufacture of a medicament in ophthalmic surgery

12. Use according to the preceding claim, **characterized in that** the ophthalmic surgery is a macular surgery and/or cataract surgery.

13. Use according to one of claims 11 and 12 for the manufacture of a medicament for staining of the internal limiting membrane and/or the lens capsule of the eye in ophthalmic surgery.

14. Use according to one of claims 11 to 13, **characterized in that** the fluorescent dye is Rhodamine.

15. Use according to one of claims 11 to 14 for the manufacture of a medicament in ophthalmic surgery of a mammal.

16. Use according to the preceding claim, **characterized in that** the mammal is a human.
